# EUROPÄISCHE PATENTSCHRIFT

(11) **EP 0 704 429 B1**
(45) Veröffentlichungstag und Bekanntmachung des Hinweises auf die Patenterteilung: **10.06.1998**
(21) Anmeldenummer: 95112399.1
(22) Anmeldetag: 07.08.1995
(51) Int. Cl.: C07C 255/61, C07C 253/30, C07D 239/42

(54) **2-(Arylimino-methyl)-3-(di-substituierte-amino)-alcylnitrile, Verfahren zu ihrer Herstellung und ihre Verwendung**
2-(Aryliminomethyl)-3-(disubstituted amino)acrylonitriles, a process for their preparation and their use
2-(Aryliminométhyl)-3-(amino disubstitué)acrylonitriles, un procédé pour leur préparation et leur emploi

(30) Priorität: 19.08.1994 DE 4429464
(43) Veröffentlichungstag der Anmeldung: 03.04.1996
(73) Patentinhaber: BAYER AG, 51368 Leverkusen (DE)
(72) Erfinder: Kraus, Helmut, Dr., D-51519 Odenthal (DE); Behre, Horst, Dr., D-51519 Odenthal (DE); Fiege, Helmut, Dr., D-51373 Leverkusen (DE)

(56) Entgegenhaltungen:
- DE-A- 2 005 515
- HETEROCYCLES, Bd. 36, Nr. 10, 1993 Seiten 2281-2290, JACHAK, M. ET AL. 'Synthesis with nitriles : 92. Synthesis of 5-formylcytosine derivatives'
- JOURNAL OF ORGANIC CHEMISTRY, Bd. 10, 1945 Seiten 76-86, UHLE, F.C. & JACOBS, W.A. 'The ergot alkaloids. XX. The synthesis of dihydro-dl-lysergic acid. A new synthesis of 3-substituted quinolines'

## Beschreibung

Die Erfindung betrifft N.N-disubstituierte 2-(Arylimino-methyl)-3-amino-acrylnitrile, ein Verfahren zu ihrer Herstellung durch Umsetzung von β-Anilinoacrylnitrilen mit ortho-Ameisensäureamiden und ihre Verwendung zur Herstellung von 4-Amino-5-iminiummethylen-2-pyrimidinen oder 4-Amino-5-formyl-2-pyrimidinen.

Cyanmalondialdehyd-Derivate sind wichtige Bausteine für die Herstellung verschiedener Heterocyclen. Aus ihnen lassen sich beispielsweise Formyl- und Cyanopyrimidine (JP-A 61 083 168 und 61 030 576), Cyanopyrazole (Mh. Chem. 124 (1993), 199) und Cyanopyridine (Synthesis 1987, 1124) erhalten. Besonders wichtig ist das 4-Amino-5-formyl-2-methylpyrimidin, das einen technisch wichtigen Vorläufer des 4-Amino-5-aminomethyl-2-methylpyrimidins ("Grewe-Diamin", Zwischenprodukt für die Herstellung von Vitamin B1) darstellt.

In Heterocycles 36 (10), 2281-2290 (1993) wird die Herstellung des Dianils des Cyanmalondialdehyds aus Anilin und 3-Dimethylamino-2-formyl-propennitril beschrieben. Die letztgenannte Verbindung synthetisierten die Autoren gemäß Mh. Chem. 124, 199 (1993) aus frisch hergestelltem Cyanacetaldehyd und DMF-dimethylacetal. Cyanacetaldehyd ist laut Synthesis 1992, 275 durch Ozonolyse von Allylcyanid bzw. Dicyanobuten zugänglich, gilt jedoch wegen seiner Neigung zur Selbstkondensation als recht instabil; vgl. Ann. Chem. 729, 139 (1969).

In J. Organ. Chem., Bd. 10 (1945), 76-86 wird die Herstellung des Mono- und Dianils des Cyanmalondialdehyds aus dem entsprechenden Natriumsalz beschrieben. Dieses Natriumsalz wiederum wird durch Formylierung von Cyanacetal in Gegenwart von metallischem Natrium synthetisiert.

Während diese Methoden des Standes der Technik mit instabilen Verbindungen oder sicherheitstechnisch problematischen Stoffen arbeiten, haben wir einen Weg gefunden, auf in technischem Maßstab durchführbare elegante Weise zu Verbindungen zu kommen, die als Bausteine für die Herstellung von 4-Amino-5-formyl-pyrimidinen verwendet werden können.

Gegenstand der Erfindung sind also Verbindungen der Formel 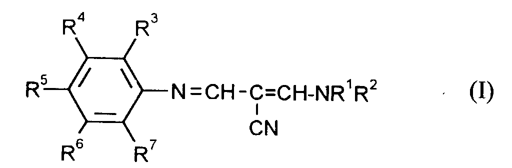 worin
- R¹ und R²: unabhängig voneinander lineares oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₇-C₁₀-Aralkyl, C₆-C₁₂-Aryl, einen 5- bis 8-gliedrigen (gesättigten oder ungesättigten) heterocyclischen Ring mit 1 bis 2 Heteroatomen aus der Reihe N, O, S oder
- R¹ und R²: gemeinsam mit dem Stickstoffatom, an dem sie sitzen, einen 5- bis 8-gliedrigen Ring, der ein weiteres Heteroatom aus der Reihe N, O, S enthalten kann,
- R³ bis R⁷: unabhängig voneinander lineares oder verzweigtes C₁-C₈-Alkyl, C₁-C₆-Alkoxy, C₁-C₆-Alkylthio, C₂-C₈-Alkenyl, C₂-C₈-Alkoxyalkyl, C₃-C₈-Cycloalkyl, Halogen, Di-C₁-C₆-alkylamino, vorzugsweise Wasserstoff, bedeuten.

Weiterer Gegenstand der Erfindung ist ein Verfahren zur Herstellung der Verbindungen I durch Umtsetzung von Verbindung der Formel 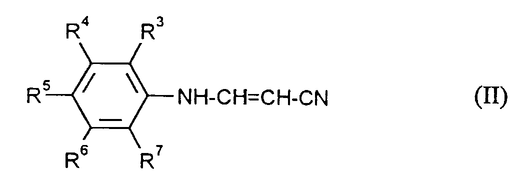 worin
- R³ bis R⁷: die obigen Bedeutungen besitzen,
mit Verbindungen der Formel 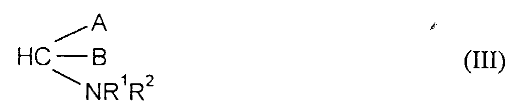 worin
- R¹ und R²: die obigen Bedeutungen, besitzen und
- A und B: unabhängig voneinander OR⁸, OR⁹, NR¹⁰R¹¹ oder NR¹²R¹³ bedeuten, worin die Substituenten R⁸ bis R¹³ unabhängig voneinander die oben für R¹ und R² definierten Bedeutungen besitzen,
in einem Molverhältnis II/III von 0,5 bis 20, vorzugsweise 0,8 bis 5, insbesondere 1 bis 3, bei einer Temperatur von 0 bis 200°C, vorzugsweise 20 bis 150°C.

Der Druck hängt von der Reaktionstemperatur und der Art der Verbindung III, insbesondere vom Siedepunkt der Produkte HA und HB ab und kann 0,1 bis 20, vorzugsweise 1 bis 5 bar betragen.

Die Zugänglichkeit der Verbindung I nach dem erfindungsgemäßen Verfahren ist überaus überraschend. Umsetzungsprodukte von Anilin und Acetaldehyd liegen nämlich nicht in der Form 1, sondern in der Form 2 vor 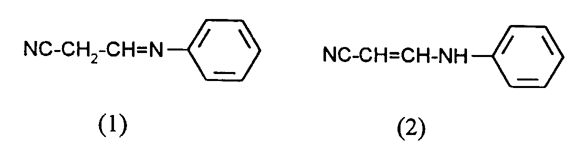 (M. Jachak et al., Monatshefte für Chemie 124, 199 (1993) in Verbindung mit H.-J. Sturm et al., Liebigs Ann. Chem. 729, 139 (1969)). Dementsprechend hätte man erwarten müssen, daß bei der Umsetzung mit Verbindung III eine Alkylierung am Stickstoff stattfinden sollte; R.F. Abdulla et al., Tetrahedron 35, 1675 ff. (1979).

Lineares und verzweigtes C₁-C₈-Alkyl umfaßt beispielsweise Methyl, Ethyl, Propyl, Isopropyl, Butyl, Isobutyl, tert.-Butyl, eines der isomeren Pentyle, Hexyle oder Octyle, bevorzugt die genannten C₁-C₄-Alkylreste.

Lineares oder verzweigtes C₂-C₈-Alkenyl umfaßt beispielsweise Vinyl, Propenyl, Allyl, eines der isomeren Butenyle, Pentenyle, Hexenyle oder Octenyle, bevorzugt die genannten C₃-C₄-Alkenylreste.

Lineares oder verzweigtes C₂-C₈-Alkoxyalkyl umfaßt beispielsweise Methoxymethyl, Ethoxymethyl sowie weitere Reste aus der Gruppe C₃-C₉-Alkyl, in welchem eine CH₂-Gruppe durch ein O-Atom ersetzt ist.

Lineares oder verzweigtes C₃-C₈-Alkoxyalkenyl umfaßt beispielsweise Methoxyvinvl, Ethoxyvinyl, Methoxyallyl, 2-Methoxy-propenyl sowie weitere Reste aus der Gruppe C₄-C₉-Alkenyl, worin eine CH₂-Gruppe durch ein O-Atom ersetzt ist. C₃-C₈-Cycloalkyl umfaßt beispielsweise Cyclopropyl, Methylcyclopropyl, Dimethyl-cyclopropyl, Cyclobutyl, Methylcyclobutyl, Cyclopentyl, Methylcyclopentyl, Cyclohexyl, Methyl-cyclohexyl, Dimethyl-cyclohexyl, Cycloheptyl, Cyclooctyl, bevorzugt Cyclopropyl und Cyclohexyl sowie deren Methyl- und Dimethyl-Derivate.

C₇-C₁₀-Aralkyl umfaßt beispielsweise 1-Phenylethyl, 2-Phenylethyl, bevorzugt Benzyl.

Als 5- bis 8-gliedriger gesättigter oder ungesättigter heterocyclischer Ring mit 1 bis 2 Heteroatomen aus der Gruppe N, O, S seien genannt: Pyrrol, Furan, Thiophen, Pyrrolidin, Pyrrolin, Pyrazol, Imidazol, Thiazol, Oxazol, Pyridin, Pyrimidin, Piperazin, Morpholin, Pyran, Azepin, Azocin, Isoxazol, Isothiazol, Pyridazin und Pyrazin, die am N-Atom durch C₁-C₄-Alkyl oder durch Hydroxy-C₁-C₄-alkyl substituiert sein können. Die heterocyclischen Ringe Morpholin, Pyrrolidin und Piperidin, die durch C₁-C₄-Alkyl oder durch Hydroxy-C₁-C₄-alkyl substituiert sein können, sind besonders bevorzugt.

R¹ und R² bedeuten unabhängig voneinander vorzugsweise lineares oder verzweigtes C₁-C₈-Alkyl, Cyclopropyl, Cyclopentyl, Cyclohexyl, Phenyl, Benzyl oder zusammen mit dem Stickstoffatom, an dem sie sitzen, einen 5- oder 6-gliedrigen Ring, der ein weiteres Heteroatom aus der Reihe N, O, S enthalten kann.

R¹ und R² bedeuten unabhängig voneinander insbesondere lineares oder verzweigtes C₁-C₄-Alkyl oder gemeinsam mit dem Stickstoffatom, an dem sie sitzen, Morpholin, Pyrrolidin oder Piperidin, die durch C₁-C₄-Alkyl oder Hydroxy-C₁-C₄-alkyl substituiert sein können.

Die Edukte II, wie beispielsweise 3-Phenylaminoacrylnitril, können z.B. aus Anilin, 3-Ethoxyacrylnitril und Ammoniak gemäß EP-A 18 473 oder in analoger Weise hergestellt werden.

Auch Dialkylaminoacrylnitrile, die technisch einfach und in nahezu quantitativer Ausbeute beispielsweise analog EP-A 560 158 zugänglich sind, lassen sich mit Arylaminen unter Säurezusatz zu den Edukten II umsetzen.

Die Edukte III umfassen Dimethylformamidacetale, Aminalester und Triaminomethane der Formeln

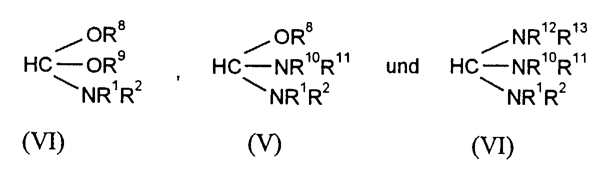

Die erfindungsgemäßen Reaktionen können beispielhaft wie folgt dargestellt werden:

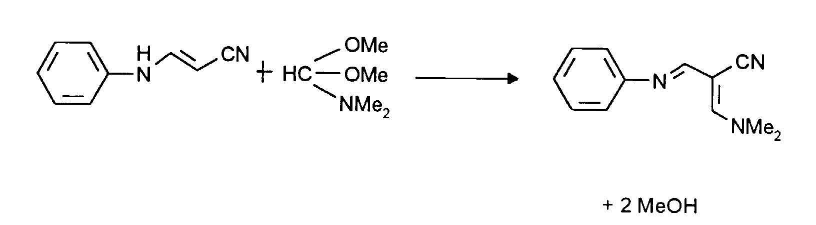

Das erfindungsgemäße Verfahren kann in An- oder Abwesenheit eines organischen Lösungsmittels durchgeführt werden. Bevorzugte organische Lösungsmittel sind aprotische und polare Lösungsmittel, beispielsweise N-persubstituierte Säureamide, wie Dimethylformamid (DMF), Dimethylacetamid (DMAC), Diethylacetamid und deren Homologe, N-Methyl-pyrrolidon (NMP), N-Methyl-caprolactam (NMC), Phosphorsäure-hexamethyltriamid, Tetramethyl-harnstoff und ähnliche, die Gruppe des Sulfolans und seiner durch Methyl, Ethyl und andere inerte Substituenten substituierten Derivate, die Gruppe der Sulfoxide wie Dimethylsulfoxid, Diethylsulfoxid und andere, die Gruppe der Sulfone wie Diethylsulfon, Dimethylsulfon und andere. Da auch o-Amide polaren aprotischen Charakter haben, ist es möglich, einen Überschuß der Verbindungen III als systemeigene Lösungs- und Verdünnungsmittel einzusetzen. Es ist sogar möglich, auf systemfremde aprotische polare Lösungsmittel völlig zu verzichten. Für den Fall, daß ein Fremdlösungsmittel eingesetzt wird, sind N-persubstituierte Säureamide, speziell NMP, NMC, DMF oder Tetramethyl-harnstoff, ganz besonders bevorzugt.

Das Fortschreiten der Reaktion kann beispielsweise dünnschichtchromatographisch verfolgt werden. Nach Beendigung der Reaktion können Lösungsmittel (falls vorhanden) und die (meist) leichtflüchtigen Alkohole bzw. Amine HA und HB abgezogen werden. Da die Reaktion sehr selektiv verläuft, hat das Rohprodukt bereits oft eine Reinheit von über 95 % und kann so beispielsweise für Heterocyclensynthesen direkt verwendet werden.

Weiterer Gegenstand der Erfindung ist die Verwendung der Verbindungen I zur Herstellung von 4-Amino-5-iminiummethylen-2-pyrimidinen oder 4-Amino-5-formyl-2-pyrimidinen. Zu diesem Zweck können die Verbindungen I mit C₁-C₆-Amidinen bei erhöhter Temperatur, z.B. bei 60 bis 150°C, zu den 4-Amino-5-iminiummethylen-2-pyrimidinen umgesetzt werden, woraus im Falle wäßriger Aufarbeitung durch Hydrolyse die entspechenden 5-Formylverbindungen entstehen.

Die Prozentangaben der folgenden Beispiele beziehen sich jeweils auf das Gewicht.

### Beispiele

### Beispiel 1

29 g trans-3-Ethoxyacrylnitril, 20,5 g Ammoniak, 21 g Anilin und 10 ml Ethanol wurden in einem 0,3 l V4A-Autoklaven 4 h bei 100°C gerührt. Nach Abdestillation des Ethanols wurde mit 200 ml Wasser versetzt und 3 mal mit 100 ml Diethylether extrahiert. Die nach Einengen erhaltenen 33 g Rückstand wurden aus Chloroform umkristallisiert, was 15,2 g trans-3-Phenylaminoacrylnitril ergab. Die Identifikation erfolgte durch GC/MS-Analyse und Vergleich der NMR-Daten mit denen aus EP-A 18 473.

¹H-NMR (d-DMSO): 4,60 ppm (d,1H); 6,9-7,3 (m,5H); 7,85 (dd, 1H); 9,70 (d,1H)

### Beispiel 2

405 g Acetonitril und 913 g 89,6 %iges Ethoxy-bis(dimethylamino)methan (Rest DMF) wurden in einem 3 l V4A-Autoklaven 12 h auf 140°C erhitzt. Nach Destillation wurde 3-Dimethylaminoacrylnitril als 98,8 %ige Ware in 96,7 % der Theorie erhalten.

29 g 3-Dimethylaminoacrylnitril, 28 g Anilin und 20 g Eisessig in 100 ml Dichlorethan wurden 17 h bei Raumtemperatur gerührt.

Anschließend wurde Wasser im Volumen-Verhältnis 1:1 zugesetzt, die Phasen getrennt und die wäßrige Phase mit Dichlorethan extrahiert. Nach dem Einengen der vereinigten organischen Medien erhielt man 40 g Rohprodukt, das langsam durchkristallisierte.

Laut ¹H-NMR waren 90 % trans-3-Phenylaminoacrylnitril, 5 % cis-Verbindung und 3 % Dimethylaminomethylenglutaconsäuredintiril vorhanden.

¹H-NMR der cis-Verbindung (d-DMSO): 4,27 ppm (d, 1H); 7,50 (dd, 1H); 6,9-7,3 (m, 5H); 9,52 ppm (d, 1H).

### Beispiel 3

14,4 g trans-3-Phenylaminoacrylnitril, 20 ml 82 %iges DMF-dimethylacetal (enthielt DMF und Methanol) und 150 ml DMF wurden 2 h am Rückfluß gekocht. Nach dem Einengen am Rotationsverdampfer wurden 20,0 g 97,8 %iges 3-Phenylimino-2-dimethylaminopropen-2-nitril entsprechend 98,3 % der Theorie erhalten.

¹H-NMR (d-DMSO): 3,18 und 3,31 ppm (breite s, je 3H, NMe₂); 6,95-7,10 und 7,25-7,35 (m, 5H, Phenyl), 7,60 (s, 1H).

## Patentansprüche

1. Verbindungen der Formel 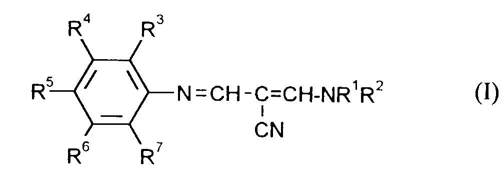 worin
R¹ und R² unabhängig voneinander lineares oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₇-C₁₀-Aralkyl, C₆-C₁₂-Aryl, einen 5- bis 8-gliedrigen (gesättigten oder ungesättigten) heterocyclischen Ring mit 1 bis 2 Heteroatomen aus der Reihe N, O, S oder
R¹ und R² gemeinsam mit dem Stickstoffatom, an dem sie sitzen, einen 5-bis 8-gliedrigen Ring, der ein weiteres Heteroatom aus der Reihe N, O, S enthalten kann,
R³ bis R⁷ unabhängig voneinander lineares oder verzweigtes C₁-C₈-Alkyl, C₁-C₆-Alkoxy, C₁-C₆-Alkylthio, C₂-C₈-Alkenyl, C₂-C₈-Alkoxyalkyl, C₃-C₈-Cycloalkyl, Halogen, Di-C₁-C₆-alkylamino, vorzugsweise Wasserstoff, bedeuten.

2. Verfahren zur Herstellung der Verbindungen I nach Anspruch 1 durch Umsetzung von Verbindungen der Formel 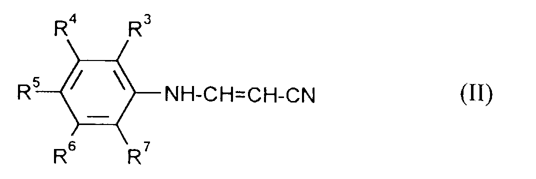 worin
R³ bis R⁷ die in Anspruch 1 angegebenen Bedeutungen besitzen,
mit Verbindungen der Formel 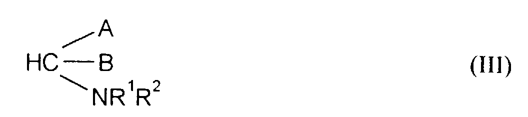 worin
R¹ und R² die in Anspruch 1 angegebenenen Bedeutungen besitzen und
A und B unabhängig voneinander OR⁸, OR⁹, NR¹⁰R¹¹ oder NR¹²R¹³ bedeuten, worin die Substituenten R⁸ bis R¹³ unabhängig voneinander die oben für R¹ und R² definierten Bedeutungen besitzen,
in einem Molverhältnis II/III von 0,5 bis 20 bei einer Temperatur von 0 bis 200°C.

3. Verfahren nach Anspruch 2 durch Umsetzung der Verbindungen II und III im Molverhältnis von 0,8 bis 5.

4. Verfahren nach Anspruch 2 durch Umsetzung der Verbindungen II und III im Molverhältnis von 1 bis 3.

5. Verfahren nach Ansprüchen 2 bis 4 bei einer Temperatur von 20 bis 150°C.

6. Verwendung der Verbindungen nach Anspruch 1 zur Herstellung von 4-Amino-5-iminiummethylen-2-pyrimidinen oder 4-Amino-5-formyl-2-pyrimidinen durch Umsetzung mit C₁-C₆-Amidinen bei erhöhter Temperatur und gegebenenfalls nachfolgende Hydrolyse.

## Claims

1. Compounds of the formula 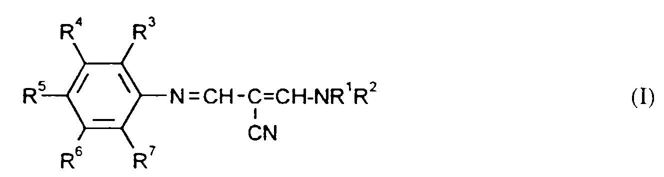 wherein
R¹ and R² independently of one another denote linear or branched C₁-C₈-alkyl, C₂-C₈-alkenyl, C₂-C₈-alkoxyalkyl, C₃-C₈-alkoxyalkenyl, C₃-C₈-cycloalkyl, C₇-C₁₀-aralkyl, C₆-C₁₂-aryl or a 5- to 8-membered (saturated or unsaturated) heterocyclic ring having 1 or 2 heteroatoms from the series consisting of N, O and S, or
R¹ and R², together with the nitrogen atom on which they are located, denote a 5- to 8-membered ring which can contain a further heteroatom from the series consisting of N, O and S and
R³ to R⁷ independently of one another denote linear or branched C₁-C₈-alkyl, C₁-C₆-alkoxy, C₁-C₆-alkylthio, C₂-C₈-alkenyl, C₂-C₈-alkoxyalkyl, C₃-C₈-cycloalkyl, halogen, di-C₁-C₆-alkylamino or, preferably, hydrogen.

2. Process for the preparation of compounds I according to Claim 1 by reaction of compounds of the formula 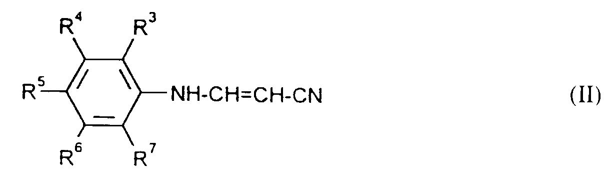 wherein
R³ to R⁷ have the meanings given in Claim 1,
with compounds of the formula 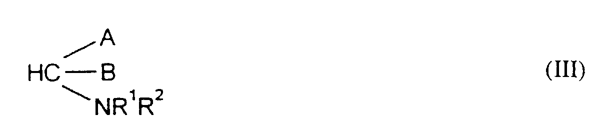 wherein
R¹ and R² have the meanings given in Claim 1 and
A and B independently of one another denote OR⁸, OR⁹, NR¹⁰R¹¹ or NR¹²R¹³, wherein the substituents R⁸ to R¹³ independently of one another have the meaning defined above for R¹ and R²,
in a molar ratio of II/III of 0.5 to 20, at a temperature of 0 to 200°C.

3. Process according to Claim 2 by reaction of the compounds II and III in a molar ratio of 0.8 to 5.

4. Process according to Claim 2 by reaction of the compounds II and III in a molar ratio of 1 to 3.

5. Process according to Claims 2 to 4 at a temperature of 20 to 150°C.

6. Use of the compounds according to Claim I for the preparation of 4-amino-5-iminiummethylene-2-pyrimidines or 4-amino-5-formyl-2-pyrimidines by reaction with C₁-C₆-amidines at elevated temperature and, if appropriate, subsequent hydrolysis.

## Revendications

1. Composés de formule 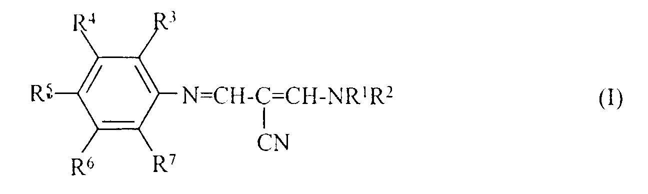 dans laquelle
R¹ et R² représentent chacun, indépendamment l'un de l'autre, un groupe alkyle en C₁-C₈, alcényle en C₂-C₈, alcoxyalkyle en C₂-C₈, alcoxyalcényle en C₃-C₈, à chaîne droite ou ramifiée, un groupe cycloalkyle en C₃-C₈, aralkyle en C₇-C₁₀, aryle en C₆-C₁₂, un hétérocycle de 5 à 8 chaînons (saturé ou insaturé) contenant 1 à 2 hétéroatomes choisis parmi N, O, S, ou bien
R¹ et R² représentent ensemble et avec l'atome d'azote auquel ils sont reliés un cycle de 5 à 8 chaînons qui peut contenir un autre hétéroatome choisi parmi N, O, S,
R³ à R⁷ représentent chacun, indépendamment les uns des autres, un groupe alkyle en C₁-C₈, alcoxy en C₁-C₆, alkylthio en C₁-C₆, alcényle en C₂-C₈, alcoxyalkyle en C₂-C₈ à chaîne droite ou ramifiée, un groupe cycloalkyle en C₃-C₈, un halogène, un groupe di(alkyle en C₁-C₆)amino, de préférence l'hydrogène.

2. Procédé de préparation des composés I de la revendication 1 par réaction de composés de formule 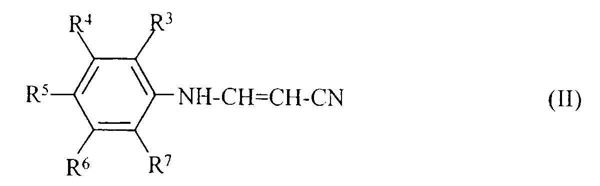 dans laquelle
R³ à R⁷ ont les significations indiquées dans la revendication 1, avec des composés de formule 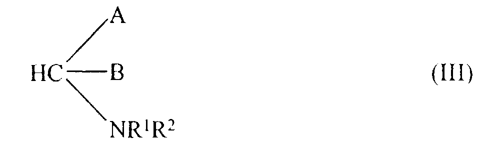 dans laquelle
R¹ et R² ont les significations indiquées dans la revendication 1 et
A et B représentent chacun, indépendamment l'un de l'autre, OR⁸, OR⁹, NR¹⁰R¹¹ ou NR¹²R¹³, les symboles R⁸ à R¹³ ayant chacun, indépendamment les uns des autres, l'une des significations indiquées ci-dessus pour R¹ et R², à un rapport molaire II/III de 0,5 à 20 et des températures de 0 à 200°C.

3. Procédé selon la revendication 2, par réaction des composés II et III à un rapport molaire de 0,8 à 5.

4. Procédé selon la revendication 2, par réaction des composés II et III à un rapport molaire de 1 à 3.

5. Procédé selon les revendications 2 à 4, à une température de 1 à 150°C.

6. Utilisation des composés selon la revendication 1 pour la préparation des 4-amine-5-iminium-méthylène-2-pyrimidines ou des 4-amino-5-formyl2-pyrimidines par réaction avec des amidines en C₁ à C₆ à température élevée, suivie le cas échéant d'une hydrolyse.
